(19) Europäisches Patentamt
European Patent Office
Office européen des brevets

(11) Veröffentlichungsnummer: **0 197 189**
**A2**

(12) # EUROPÄISCHE PATENTANMELDUNG

(21) Anmeldenummer: 85113728.1

(22) Anmeldetag: 29.10.85

(51) Int. Cl.⁴: C 07 D 285/12
C 07 D 417/12, A 61 K 31/41

(30) Priorität: 12.04.85 DE 3513184

(43) Veröffentlichungstag der Anmeldung:
15.10.86 Patentblatt 86/42

(84) Benannte Vertragsstaaten:
AT BE CH DE FR GB IT LI LU NL SE

(71) Anmelder: HEUMANN PHARMA GMBH & CO
18-28 Heideloffstrasse
D-8500 Nürnberg(DE)

(72) Erfinder: Schickaneder, Helmut, Dr. Dipl.-Chem.
Moosäcker 25
D-8501 Eckental(DE)

(72) Erfinder: Herter, Rolf, Dr. Dipl.-Chem.
Bayernstrasse 42
D-8540 Schwabach(DE)

(72) Erfinder: Wegner, Kurt, Dr.
Gänsmarkt 3
D-6500 Mainz 3(DE)

(72) Erfinder: Schunack, Helmut, Prof. Dr. Dr. Dipl.-Chem.
Spanische Allee 95
D-1000 Berlin 38(DE)

(72) Erfinder: Szelenyi, Istvan, Dr.
Haendelstrasse 32
D-8501 Schwaig(DE)

(72) Erfinder: Postius, Stefan, Dr.
Nunnenbeckstrasse 24
D-8500 Nürnberg 20(DE)

(72) Erfinder: Ahrens, Kurt Henning, Dr.
Praterstrasse 9
D-8500 Nürnberg 20(DE)

(74) Vertreter: Kraus, Walter, Dr. et al,
Patentanwälte Kraus, Weisert & Partner
Thomas-Wimmer-Ring 15
D-8000 München 22(DE)

(54) **1,3,4-Thiadiazolderivate, Verfahren zu ihrer Herstellung und diese Verbindungen enthaltende Arzneimittel.**

(57) Es werden 1,3,4-Thiadiazolderivate der allgemeinen Formel I

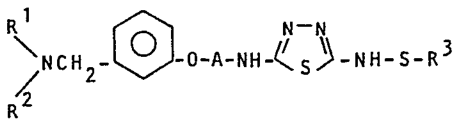

(I)

beschrieben, welche hochwirksame Hemmstoffe für Histamin-$H_2$-Rezeptoren darstellen. Diese Vervindungen besitzen zusätzlich eine cytroprotektive Wirkung.

EP 0 197 189 A2

# 1,3,4-Thiadiazolderivate, Verfahren zu ihrer Herstellung und diese Verbindungen enthaltende Arzneimittel

Die Erfindung betrifft neue 1,3,4-Thiadiazolderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Antiulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 bis 300 mg in einer therapeutisch festgelegten Form zu verabreichen. Es besteht somit ein Bedarf an Antiulcusmitteln, die wirksamer sind als Cimetidin und Ranitidin.

Der Erfindung liegt die Aufgabe zugrunde, neue hochwirksame Hemmstoffe für Histamin-$H_2$-Rezeptoren mit zusätzlicher cytoprotektiver Wirkung zur Verfügung zu stellen. Diese Aufgabe wird durch die erfindungsgemäßen Verbindungen gelöst.

Die erfindungsgemäßen Verbindungen zeigen aufgrund ihrer spezifischen $H_2$-antagonistischen Aktivität eine Hemmung der Magensäuresekretion, wenn diese durch Histaminagonisten stimuliert wird [Ash und Schild, "Brit. J. Pharmacol. Chemother.", 27, 427 (1966) und Black et al., "Nature", 236, 385 (1971)]. Weiterhin zeigen die erfindungsgemäßen Verbindungen eine zusätzliche cytoprotektive Wirkung. Die pharmakologische Aktivität dieser Verbindungen kann nach einer modifizierten Methode gemäß der DE-OS 27 34 070 beim perfundierten Rattenmagen gezeigt werden oder durch die Ermittlung der $pA_2$-Werte in vitro am Meerschweinchenvorhof (vgl. Ariens, "Molecular Pharmacology", Band 1, Academic Press, New York, 1964) nachgewiesen werden. Weiterhin kann die $H_2$-antagonistische Wirkung an wachen Heidenhain-Pouch-Hunden nach der Methode von Black et al., "Nature", 236, 385 (1971) gezeigt werden. Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Methacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Gegenstand der Erfindung sind neue 1,3,4-Thiadiazolderivate der allgemeinen Formel I

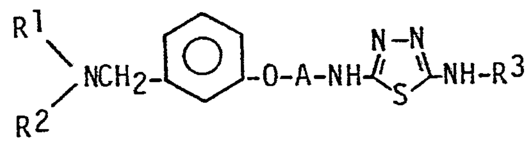

(I)

in der $R^1$ und $R^2$, die gleich oder verschieden sein können, jeweils für Wasserstoff, lineares $C_1$-$C_{10}$-Alkyl, Polycycloalkyl, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen 5-bis 10gliedrigen stickstoffhaltigen alicyclischen, heterocyclischen Ring bilden, A die Gruppierung $-(CH_2)_n-$ oder $-CH_2-CH=CH-CH_2-$ bedeutet, wobei n den Wert 3 oder 4 hat, $R^3$ für lineares oder verzweigtkettiges $C_1$-$C_6$-Alkyl oder $C_5$-$C_6$-Cycloalkyl oder für unsubstituiertes oder ein- bis dreifach substituiertes Aryl oder Heteroaryl steht, sowie die physiologisch annehmbaren Salze davon.

In der allgemeinen Formel I bedeuten die Substituenten $R^1$ und $R^2$, die gleich oder verschieden sein können, jeweils ein Wasserstoffatom, eine lineare $C_1$-$C_{10}$-Alkylgruppe, vorzugsweise eine lineare $C_1$-$C_6$-Alkylgruppe, ganz besonders bevorzugt eine lineare $C_1$-$C_3$-Alkylgruppe, zum Beispiel eine Methyl-, Ethyl- oder Propylgruppe, eine $C_5$-$C_6$-Cycloalkylgruppe, d. h. eine Cyclopentyl- oder Cyclohexylgruppe, eine $C_1$-$C_3$-Alkylaminogruppe, zum Beispiel eine n- oder i-Propylaminogruppe, eine Ethylaminogruppe oder eine Methylaminogruppe, wobei die Methylaminogruppe bevorzugt wird, oder eine Di($C_1$-$C_3$-alkyl)aminogruppe, vorzugsweise eine Diethyl- oder Dimethylaminogruppe. $R^1$ und $R^2$ können aber auch zusammen mit dem Stickstoffatom, an das sie gebunden sind, einen gegebenenfalls ein- oder

0197189

zweifach mit einer $C_1$-$C_3$-Alkylgruppe substituierten 5- bis 10gliedrigen stickstoffhaltigen alicyclischen, heterocyclischen Ring bilden. Dieser Ring kann gegebenenfalls ein oder mehrere weitere Heteroatome, zum Beispiel ein weiteres Stickstoffatom, ein Sauerstoffatom oder ein Schwefelatom, enthalten. Bevorzugte Beispiele für den so definierten 5- bis 10-gliedrigen heterocyclischen Ring sind der Pyrrolidin-, Piperidin- und Homopiperidinring. Bevorzugte Beispiele für heterocyclische Ringe, die weitere Heteroatome enthalten, sind der Piperazin- und der Morpholinring. Im Falle einer Ein- oder Zweifachsubstitution am 5- bis 10gliedrigen stickstoffhaltigen alicyclischen, heterocyclischen Ring bedeuten die Substituenten jeweils eine $C_1$-$C_3$-Alkylgruppe, vorzugsweise eine Methylgruppe. So kann zum Beispiel der Pyrrolidin-, Piperidin- oder Homopiperidinring jeweils in 2-, 3- oder 4-Stellung oder bei einer Zweifachsubstitution in 2,5- oder 2,6-Stellung mit einer Methylgruppe substituiert sein. Bevorzugte substituierte stickstoffhaltige 5- bis 10gliedrige alicyclische, heterocyclische Ringe sind zum Beispiel der 3-Methylpyrrolidin-, 3-Methylpiperidin-, 4-Methylpiperidin- oder 3,5-Dimethylpiperidinring, ganz besonders bevorzugt sind der 3-Methylpyrrolidin- und 3-Methylpiperidinring.

A bedeutet eine der folgenden Gruppierungen: $-(CH_2)_n-$ oder $-CH_2-CH=CH-CH_2-$, n bedeutet eine ganze Zahl 3 oder 4, wobei der Wert 3 bevorzugt wird.

$R^3$ bedeutet lineares oder verzweigtkettiges $C_1$-$C_6$Alkyl, vorzugsweise $C_1$-$C_3$-Alkyl, wie zum Beispiel Methyl, Ethyl, Propyl, Isopropyl, Butyl, Isobutyl, n-Pentyl, Isopentyl, n-Hexyl und Isohexyl, oder $C_5$-$C_6$-Cycloalkyl, wie Cyclopentyl oder Cyclohexyl.

Eine weitere Bedeutung für $R^3$ ist unsubstituiertes oder ein- bis dreifach substituiertes Aryl. Beispiele für Aryl sind Phenyl oder Naphthyl, wobei Phenyl bevorzugt wird. Der Arylrest kann ein- bis dreifach substituiert sein, und zwar insbesondere durch Halogen, wie Chlor und Brom, lineares oder verzweigtkettiges Niedrigalkyl, vorzugsweise $C_1$-$C_4$-Alkyl, insbesondere Methyl, Niedrigalkoxy, Trifluormethyl und Nitro. Hierin werden unter "Niedrigalkyl" bzw. "Niedrigalkoxy" etc. Gruppen mit 1 bis

6, vorzugsweise 1 bis 4, Kohlenstoffatomen im Alkylteil verstanden. Bei Einfachsubstitution kann der Substituent in ortho-, meta- oder para-Position, vorzugsweise in para-Position, gebunden sein. Bei der Zweifachsubstitution sind die ortho-, para- (2,4-Stellung am Phenylring) bzw. die ortho-, ortho- (2,6-Stellung am Phenylring) Positionen bevorzugt.

Eine weitere Bedeutung für $R^3$ ist unsubstituiertes oder ein- bis dreifach substituiertes Heteroaryl. Beispiele für solche Heteroarylgruppen sind Pyridyl, Pyrimidinyl, Thiazolyl und Benzothiazolyl, wobei Pyridyl und Pyrimidinyl bevorzugt werden. Die Heteroarylgruppen können unsubstituiert sein oder mit den gleichen Gruppen wie oben für Aryl beschrieben und auf die gleiche Weise wie oben für Aryl beschrieben ein- bis dreifach substituiert sein. Bevorzugt wird hierbei eine einfache Substitution mit einer Niedrigalkylgruppe, insbesondere einer $C_1$-$C_4$-Alkylgruppe.

Die erfindungsgemäßen Verbindungen können durch ein Verfahren hergestellt werden, bei dem man ein Amin der allgemeinen Formel II

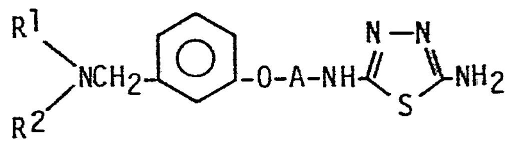

(II)

in der $R^1$, $R^2$ und A die oben angegebenen Bedeutungen besitzen, in einem inerten Lösungsmittel, wie Ether, Tetrahydrofuran, Dioxan oder Dimethylformamid, vorzugsweise Tetrahydrofuran, mit vorzugsweise äquimolaren Mengen eines Sulfenylhalogenids der allgemeinen Formel III

$$R^3\text{-S-Hal} \qquad \text{(III)}$$

worin Hal für ein Chlor- oder Bromatom, vorzugsweise Chloratom, steht und $R^3$ die oben angegebene Bedeutung hat, in einer basenkatalysierten Reaktion bei einer Temperatur von 0°C bis 20°C, vorzugsweise bei 5°C, umsetzt. Als Base lassen sich tertiäre Amine, wie Dimethylbenzylamin, Triethylamin oder Pyridin, verwenden. Die Aufarbeitung erfolgt in an sich bekannter Weise, beispielsweise durch Einengen des Reaktionsgemisches, Kristallisation und/oder Reinigung durch Säulenchromatographie.

Die Herstellung des Sulfenylhalogenids der allgemeinen Formel III erfolgt nach bekannten Literaturverfahren (siehe u.a. E. Kühle, "The Chemistry of Sulfenic Acids", Georg Thieme Verlag (1973)).

Die Amine der allgemeinen Formel II werden in an sich bekannter Weise hergestellt. So kann man zum Beispiel eine Verbindung der allgemeinen Formel VI

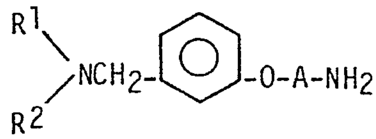
(VI)

in der $R^1$, $R^2$ und A die oben angegebenen Bedeutungen besitzen, mit einem 1,3,4-Thiadiazol der Formel VII

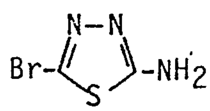
(VII)

(Herstellung analog G. Werber et al., "J. Heterocycl. Chem.", 14, 823 (1977)) in einem inerten Lösungsmittel, wie Tetrahydrofuran oder Dioxan, in einer basenkatalysierten Reaktion umsetzen. Als Base verwendet man vorzugsweise Triethylamin.

Die erfindungsgemäßen Verbindungen können weiterhin durch ein Verfahren hergestellt werden, bei dem man eine Verbindung der allgemeinen Formel IV

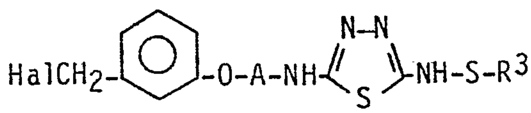
(IV)

worin Hal für ein Chlor- oder für ein Bromatom steht und A und $R^3$ die oben angegebenen Bedeutungen besitzen, in einem inerten Lösungsmittel, wie zum Beispiel Tetrahydrofuran oder Dioxan, mit vorzugsweise äquimolaren Mengen eines Amins der allgemeinen Formel V

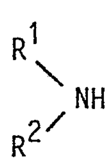
(V)

in der $R^1$ und $R^2$ die oben angegebenen Bedeutungen haben, durch eine basenkatalysierte Reaktion zu einer erfindungsgemäßen Verbindung der allgemeinen Formel I umsetzt.

Die Herstellung der Verbindung der allgemeinen Formel IV erfolgt in an sich bekannter Weise. So kann man zum Beispiel einen Alkohol der Formel VIII

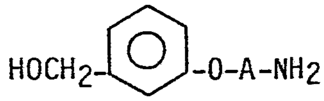
(VIII)

in der A die oben angegebene Bedeutung besitzt, mit einem 1,3,4-Thiadiazol der Formel VII

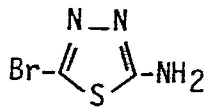
(VII)

in einem inerten Lösungsmittel, wie Tetrahydrofuran oder Dioxan, in einer basenkatalysierten Reaktion zu einer Verbindung der allgemeinen Formel IX

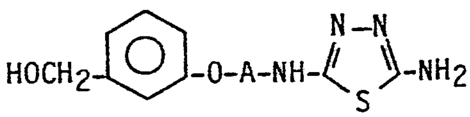
(IX)

in der A die oben angegebene Bedeutung besitzt, umsetzen, die so erhaltene Verbindung anschließend mit einem Halogenierungsmittel, wie zum Beispiel Thionylchlorid, in eine Verbindung der allgemeinen Formel X

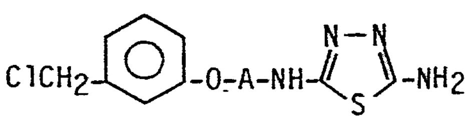
(X)

überführen und diese Verbindung X wie oben beschrieben mit vorzugsweise äquimolaren Mengen eines Sulfenylhalogenids der allgemeinen Formel III

$R^3$-S-Hal (III)

worin $R^3$ wie oben definiert ist, zu einer Verbindung der allgemeinen Formel IV umsetzen.

Die Erfindung umfaßt auch die stereoisomeren Verbindungen und die physiologisch annehmbaren Salze der genannten Verbindungen. Diese Salze können zum Beispiel mit organischen Säuren, wie Ameisensäure, Essigsäure, Propionsäure, Phenylessigsäure, Weinsäure, Zitronensäure, Fumarsäure, Methansulfonsäure, Embonsäure etc., in an sich bekannter Weise gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutisch annehmbarer Träger oder Verdünnungsmittel hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder

Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die Erfindung wird anhand der folgenden Beispiele näher erläutert.

0197189

Beispiel 1

Methode A

2-[3-[3-(Piperidinomethyl)phenoxy]propylamino]-5-pyridin-2-sulfenamido -1,3,4-thiadiazol

a) Herstellung von
N-[3-[3-(1-Piperidinomethyl)phenoxy]propyl]-1,3,4-thiadiazol-2,5-diamin

0.57 g (2.3 mmol) 1-Amino-[3-[3-(1-piperidinomethyl)phenoxy]-propan, 0.35 ml Triethylamin und 0.42 g (2.3 mmol) 5-Brom-1,3,4-thiadiazol-2- amin werden in 20 ml THF 3 Stunden unter Rückfluß erhitzt. Festes Triethylammoniumbromid wird abfiltriert, das Filtrat eingedampft und der Rückstand durch präparative Schichtchromatographie gereinigt.

Ausbeute: 0.25 g (30 % d.Th.); Schmp.: 158 - 159 °C

$C_{17}H_{25}N_5OS$ (347.5)

| | | | |
|---|---|---|---|
| Berechnet: | C 58.8 | H 7.25 | N 20.2 |
| Gefunden: | C 58.8 | H 7.28 | N 20.0 |

b) Herstellung von
2-[3-[3-(Piperidinomethyl)phenoxy)propylamino]-5-pyridin-2-sulfenamido -1,3,4-thiadiazol

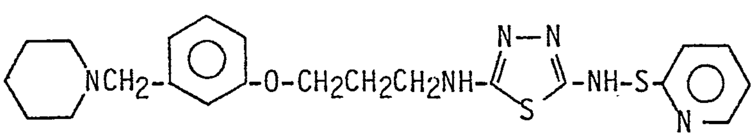

3.47 g (10 mmol) $N^2$-[3-[3-(1-Piperidinomethyl)phenoxy]propyl]-1,3,4-thiadiazol-2,5-diamin werden unter Feuchtigkeitsausschluß in 150 ml abs. Tetrahydrofuran aufgeschlämmt, mit 3.48 ml (25 mmol) abs. Triethylamin versetzt, auf 0 °C abgekühlt und tropfenweise mit einer Lösung aus 1.6 g (11 mmol) 2-Pyridinsulfensäurechlorid in 20 ml abs. 1,2-Dichlorethan umgesetzt.

Nach einer Reaktionszeit von 1 h bei Raumtemperatur wird das Reaktionsgemisch in 100 ml einer gesättigten wäßrigen $NaHCO_3$-Lösung gegossen und dreimal mit je 100 ml Ethylacetat extrahiert. Die organische Phase wird mit 50 ml einer gesättigten wäßrigen NaCl-Lösung gewaschen, über Natriumsulfat getrocknet und das Lösungsmittel im Vakuum eingeengt. Der Rückstand wird durch Säulenchromatographie (350 g $Al_2O_3$ neutral, Elutionsmittel Ethylacetat/Methanol 90/10) gereinigt.

Farblose Kristalle vom Schmelzpunkt 132 - 133 °C

Rf = 0.2 ($Al_2O_3$ neutral, Ethylacetat/Methanol 90/10)

Ausbeute: 0.46 g (10 % d.Th.)

$C_{22}H_{28}N_6OS_2$ (456.6)

| | | | | |
|---|---|---|---|---|
| Berechnet: | C 57.87 | H 6.18 | N 18.40 | S 14.04 |
| Gefunden: | C 57.71 | H 5.97 | N 18.21 | S 13.63 |

$^1$H-NMR-Spektrum: ($d_6$-DMSO, TMS als interner Standard)

$\delta$ = 1.18 - 1.63 (m) 6 H,
1.95 (m) 2 H,
2.15 - 2.42 (m) 4 H,
3.17 - 3.70 (m) 5 H
(1 H austauschbar mit $D_2O$)
4.00 (t) 2 H,
6.67 - 7.97 (m) 7 H,
8.47 (d) 1 H,
9.37(s, breit) (austauschbar mit $D_2O$) 1 H ppm.

Methode B

a) Herstellung von 3-[3-(Hydroxymethyl)phenoxy]propylamin

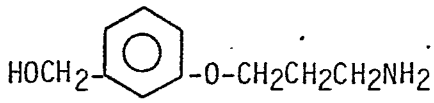

28.03 g (0.226 mol) 3-Hydroxybenzylalkohol werden mit 60.47 g (0.226 mol) Brompropylphthalimid und 5.2 g Natrium (0.226 mol) in 400 ml Ethanol unter Rückflußtemperatur (5 h) umgesetzt. Nach Abziehen des Ethanols wird mit Wasser versetzt und mit Essigsäureethylester extrahiert. Das Einengen der org. Phase ergibt 60.8 g eines hellbraunen Öls, aus dem 38 g (54 %) N-[3-[3-(Hydroxymethyl)phenoxy]propyl]phthalimid als farbloser Feststoff vom Schmelzpunkt 78 - 80° C kristallisieren. 35.3 g (113 mmol) N-[3-[3-(Hydroxymethyl)phenoxy]propyl]phthalimid werden in 300 ml Ethanol mit 13.8 ml Hydrazinhydrat (80 %) versetzt und 5 h unter Rückfluß gekocht. Das Ethanol wird im Vakuum weitgehend entfernt und der Rückstand mit 100 ml $H_2O$ und 40 ml konz. HCl versetzt (pH 1). Man saugt vom Feststoff ab, extrahiert das Filtrat mit Ethylacetat, alkalisiert mit NaOH (pH 12), sättigt mit NaCl und extrahiert mit $CH_2Cl_2/CH_3OH$ 80:20. Nach Einengen und Destillation des zurückbleibenden Öls im Kugelrohr (140 - 160° C, $5.10^{-2}$ Torr) erhält man 13.7 g (67 % d.Th.) eines hellen Feststoffes vom Schmelzpunkt 68,5 - 69° C.

| | |
|---|---|
| $^1$H-NMR-Spektrum: | $\delta$ = 1,86 (q) 2 H, |
| ($CDCl_3$, TMS als | 2,37 (s, breit) 3 H, |
| interner Standard) | 2,83 (t) 2 H, |
| | 4,00 (t) 2 H, |
| | 4,62 (s) 2 H, |
| | 6,70 - 7,57 (m) 4 H ppm. |

b) Herstellung von 2-Amino-5-[3-[3-(hydroxymethyl)phenoxy]propylamino]-1,3,4-thiadiazol.

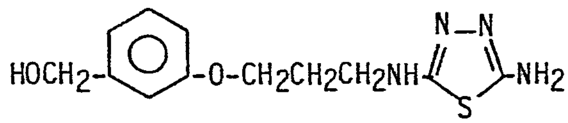

46,0 g (0.26 mol) 5-Brom-1,3,4-thiadiazol-2-amin werden mit 46,3 g (0,26 mol) 3-[3-(Hydroxymethyl)phenoxy]propylamin und 71,6 ml (0,52 mol) Triethylamin in 300 ml THF 30 h bei RT gerührt. Man versetzt mit 400 ml einer gesättigten wäßrigen Lösung von $NaHCO_3$ und extrahiert mit Essigsäureethylester. Nach dem Trocknen über $Na_2SO_4$ engt man im Vakuum ein und kristallisiert den Rückstand aus Ethanol/Essigsäureethylester.

Ausbeute: 45,7 g (63 % d.Th.)

Schmelzpunkt: 94 - 95° C

Rf: 0,51 (Dichlormethan/Methanol 80/20)

| | | | | |
|---|---|---|---|---|
| $C_{12}H_{16}N_4O_2S$ (280,3) | Ber.: | C 51,4 | H 5,75 | N 20,0 |
| | Gef.: | C 51,2 | H 5,90 | N 20,2 |

$^1$H-NMR-Daten: ($d_6$-DMSO, TMS als interner Standard)

$\delta$ = 1.97 (quin) 2 H,
3.29 (q) 2 H,
4.02 (t) 2 H,
4.46 (d) 2 H,
5.18 (t) (austauschbar mit $D_2O$) 1 H,
6.24 (s) (austauschbar mit $D_2O$) 2 H,
6.75 - 6.90 (m) 3 H,
7.22 (t) 1 H ppm.

c) Herstellung von 2-Amino-5-[3-[3-(chlormethyl)phenoxy]propylamino]-1,3,4-thiadiazol

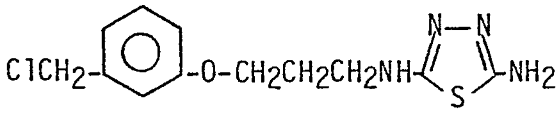

In 120 ml Thionylchlorid werden portionsweise 23,5 g (83 mmol) 2-Amino-5-[3-[3-(hydroxymethyl)phenoxy]propylamino]-1,3,4-thiadiazol unter Eiskühlung eingetragen. Man läßt auf Raumtemperatur erwärmen, rührt noch 2 h und zieht das überschüssige Thionylchlorid im Vakuum ab. Der Rückstand wird mit Wasser und Methanol versetzt, mit $NaHCO_3$ neutralisiert und mit Essigsäureethylester/Methanol 80:20 extrahiert. Nach dem Abdestillieren des Lösungsmittels im Vakuum verbleibt ein beigefarbener Feststoff, der über 500 g Kieselgel (Laufmittel: $CH_2Cl_2/CH_3OH$, 90:10) filtriert wird. Durch Einengen des Filtrats erhält man die Titelverbindung als farbloses Pulver.

Schmelzpunkt: 135 - 136° C

Ausbeute: 11,7 g (47 % d.Th.)

Rf: 0.27 ($SiO_2$, $CH_2Cl_2/CH_3OH$, 90:10)

| | | | | |
|---|---|---|---|---|
| $C_{12}H_{15}ClN_4OS$ (298.5) | Ber.: | C 48.24 | H 5.06 | N 18.75 |
| | Gef.: | C 48.30 | H 5.31 | N 19.00 |

$^1$H-NMR-Spektrum: (DMSO-$d_6$, TMS als interner Standard)

$\delta$ = 1.98 (q) 2 H,
3.33 (m) 2 H,
4.04 (t) 2 H,
4.72 (s) 2 H,
6.23 (s) 2 H (austauschbar mit $D_2O$),
6.7 - 7.6 (m) 5 H, 1 H, (austauschbar mit $D_2O$) ppm.

d) Herstellung von 5-[3-[3-Chlormethyl)phenoxy]propylamino]-2-(2-pyridinsulfenamido)-1,3,4-thiadiazol

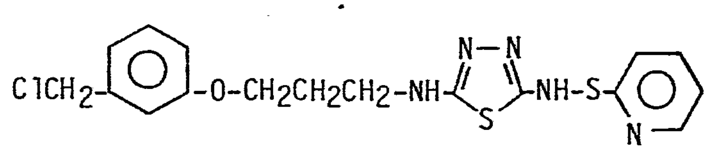

Zu 2.99 g (10 mmol) 2-Amino-5-[3-[3-(chlormethyl)phenoxy]propylamino]-1,3,4-thiadiazol in 60 ml abs. DMF tropft man eine Lösung von 2-Pyridinsulfensäurechlorid - aus 1.32 g (6 mmol) 2,2'-Dithiopyridin, 0.51 ml (6.3 mmol) $SO_2Cl_2$ - in 15 ml 1,2-Dichlormethan, wobei die Temperatur zwischen 0 und 5° C gehalten wird. Man rührt 1 h unter Eiskühlung, läßt auf Raumtemperatur erwärmen, gießt auf 100 ml einer gesättigten $NaHCO_3$-Lösung, extrahiert mit zwei 100 ml-Portionen Essigsäureethylester und trocknet die organische Phase über $Na_2SO_4$.
Nach dem Einengen im Vakuum wird der ölige Rückstand an Aluminiumoxid (Macherey-Nagel, neutral) chromatographiert (Laufmittel: Essigsäureethylester, dann Essigsäureethylester/Methanol, 90 : 10).

Ausbeute: 530 mg (13 % d.Th.)

Schmelzpunkt: 122 - 123° C

Rf: 0.30 ($Al_2O_3$ neutral, Essigsäureethylester/Methanol 90 : 10)

| | | |
|---|---|---|
| $C_{17}H_{18}ClN_5OS_2$ (407.9) | Ber.: | C 50.05 H 4.45 N 17.17 Cl 8.69 |
| | Gef.: | C 50.15 H 4.81 N 17.19 Cl 8.70 |

| | |
|---|---|
| $^1H$-NMR-Spektrum: | $\delta$ = 1.97 (q) 1 H, |
| ($d_6$-DMSO, TMS als | 3.30 (m) 2 H, |
| interner Standard) | 4.03 (t) 2 H, |
| | 4.73 (s) 2 H, |

6.8 - 7.5 (m) 7 H, 1 H (austauschbar mit $D_2O$),
7.85 (m) 1 H,
8.50 (m) 1 H,
9.37 (m) 1 H, (austauschbar mit $D_2O$) ppm.

e) Herstellung von 2-[3-[3-Piperidinomethyl)phenoxy]propylamino]-5-pyridin-2-sulfenamido-1,3,4-thiadiazol

816 mg (2 mmol) 5-[3-[3-(Chlormethyl)phenoxy]propylamino]-2-(2-pyridinsulfenamido)-1,3,4-thiadiazol werden mit 0.5 ml (5 mmol) Piperidin in 30 ml Ethanol bei Raumtemperatur 3 Tage gerührt. Man gießt dann in 100 ml einer gesättigten wäßrigen $NaHCO_3$-Lösung, extrahiert mit Essigsäureethylester und trocknet über Natriumsulfat. Nach dem Einengen im Vakuum verbleiben 830 mg eines gelben Öls, das in wenig Essigsäureethylester gelöst wird. Beim Abkühlen im Eisbad kristallisieren 290 mg (32 % d.Th.) der Titelverbindung als farbloser Feststoff aus.
Die physikalisch-chemischen Daten sind mit den unter Methode A Beispiel 1b beschriebenen identisch.

Beispiel 2

2-[3-[3-(Piperidinomethyl)phenoxy]propylamino]-5-(2-pyrimidinsulfenamido)-1,3,4-thiadiazol

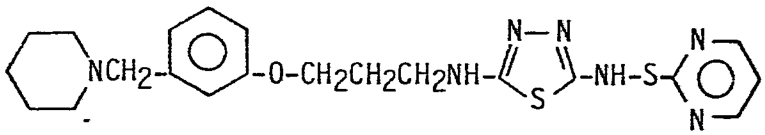

Die Herstellung erfolgt analog Beispiel 1 aus $N^2$-[3-[3-(1-Piperidinomethyl)phenoxy]propyl]-1,3,4-thiadiazol-2,5-diamin und 2-Pyrimidinsulfenylchlorid.

Hellgelbe Kristalle vom Schmelzpunkt 113 - 116 °C

Rf = 0.33 ($Al_2O_3$ basisch/Chloroform/MeOH 95/5 )

$C_{21}H_{27}N_7OS_2$ (457.6)

| | |
|---|---|
| $^1$H-NMR-Spektrum: (CDCl$_3$, TMS als interner Standard) | $\delta$ = 1.20 - 1.70 (m) 6 H,<br>2.05 (m) 2 H,<br>2.20 - 2.47 (m) 4 H,<br>3.43 (s, t) 4 H,<br>4.02 (t) 2 H,<br>6.65 - 7.35 (m) 7 H,<br>(2 H austauschbar mit $D_2O$)<br>8.57 (d) 2 H ppm. |

0197189

Beispiel 3

2-[3-[3-(Piperidinomethyl)phenoxy]propylamino]-5-benzolsulfenamido-1,3,4-thiadiazol

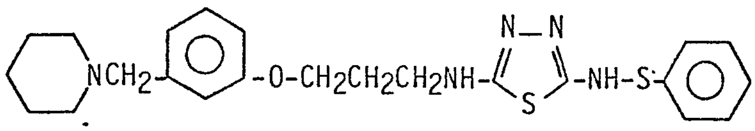

Die Herstellung erfolgt analog Beispiel 1 aus $N^2$-[3-[3-(1-Piperidinomethyl)phenoxy]propyl]-1,3,4-thiadiazol-2,5-diamin und Benzolsulfenylchlorid.

Farblose Kristalle vom Schmelzpunkt 79 - 80 °C

Rf = 0.47 ($Al_2O_3$ basisch/Essigsäureethylester/MeOH 9/1)

$C_{23}H_{29}N_5OS_2$ (455.6)

| | |
|---|---|
| $^1$H-NMR-Spektrum: (CDCl$_3$, TMS als interner Standard) | $\delta$ = 1.27 - 1.80 (m) 6 H,<br>2.17 (m) 2 H,<br>2.30 - 2.63 (m) 4 H,<br>3.53 (m) 4 H,<br>4.13 (t) 2 H,<br>5.5 - 7.0 (breit) 2 H,<br>(austauschbar mit $D_2O$)<br>6.6 - 7.4 (m) 9 H ppm. |

Beispiel 4

0197189

2-[3-[3-Piperidinomethyl)phenoxy]propylamino]-5-cyclohexylsulfenamido-1,3,4-thiadiazol

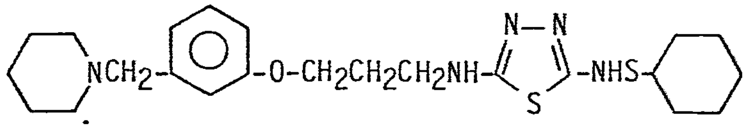

Die Herstellung erfolgt analog Beispiel 1 aus $N^2$-[3-[3-(1-Piperidinomethyl)phenoxy]propyl]-1,3,4-thiadiazol-2,5-diamin und Cyclohexylsulfenylchlorid.

Farblose Kristalle vom Schmelzpunkt 129 - 131 °C

Rf = 0.5 ($Al_2O_3$ basisch/Essigsäureethylester/MeOH 9/1)

$C_{23}H_{35}N_5OS_2$ (461.7)

| | |
|---|---|
| $^1$H-NMR-Spektrum: | $\delta$ = 1.0 - 2.5 (m) 22 H, |
| ($CDCl_3$, TMS als | 2.97 (m) 1 H, |
| interner Standard) | 3.45 (s) 2 H, |
| | 3.50 (t) 2 H, |
| | 4.09 (t) 2 H, |
| | 6.1 (breit) 2 H, (austauschbar mit $D_2O$) |
| | 6.8 - 7.4 (m) 4 H ppm. |

Beispiel 5



2-[3-[(3-Piperidinomethyl)phenoxy]propylamino]-5-tolylsulfenamido-1,3,4-thiadiazol

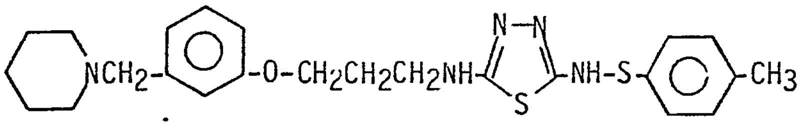

Zu 6,94 g (20 mmol) $N^2$-[3-[3-(1-Piperidinomethyl)phenoxy]propyl]-1,3,4-thiadiazol-2,5-diamin und 5,2 ml (65 mmol) Pyridin in 120 ml abs. Dimethylformamid gibt man tropfenweise bei 0 - 5° C eine Lösung von 2.37 g (15 mmol) p-Tolylsulfenylchlorid in 30 ml 1,2-Dichlorethan. Man läßt auf Raumtemperatur kommen, gießt das Reaktionsgemisch auf 200 ml einer gesättigten wäßrigen $NaHCO_3$-Lösung, extrahiert mit Essigsäureethylester, trocknet die org. Phase über $Na_2SO_4$ und filtriert. Nach dem Abziehen des Lösungsmittels im Vakuum wird der hellrote ölige Rückstand mit 150 ml Diethylether gelöst. Beim Abkühlen im Eisbad kristallisieren 3,7 g eines beigefarbenen Pulvers, das aus 140 ml Aceton umkristallisiert wird.

Ausbeute: 2,6 g (27 % d.Th.)

Farblose Kristalle vom Schmelzpunkt 142 - 143° C

Rf = 0.38 ($Al_2O_3$ basisch/Essigsäureethylester/MeOH)

$C_{24}H_{31}N_5OS_2$ (469,7)

| | |
|---|---|
| $^1$H-NMR-Spektrum: | $\delta$ = 1.40 (m) 6 H, |
| ($d_6$-DMSO, TMS als | 1.95 (m) 2 H, |
| interner Standard) | 2.28 (s) (m) 7 H, |
| | 3.30 (m) 2 H, |

3.36 (m) 3 H, 1 H (austauschbar mit $D_2O$)
3.99 (t) 2 H,
6.4 - 7.4 (m) 8 H,
9.13 (breit) 1 H (austauschbar mit $D_2O$) ppm.

Beispiel 6

2-[3-[(-Piperidinomethyl)phenoxy]-propylamino]-5-methylsulfenamido-1,3,4-thiadiazol

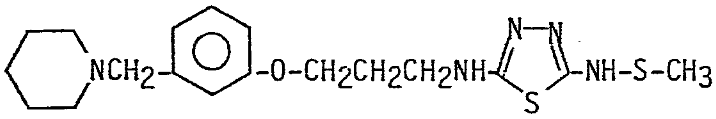

Die Herstellung erfolgt analog Beispiel 5 aus $N^2$-[3-[3-(1-Piperidinomethyl)phenoxy]propyl]-1,3,4-thiadiazol-2,5-diamin und Methylsulfenylchlorid.

Farblose Kristalle vom Schmelzpunkt 117 - 118° C

Rf = 0,43 ($Al_2O_3$ baisch/Essigsäuremethylester/MeOH)

$C_{18}H_{27}N_5OS_2$ (393,6)

$^1$H-NMR-Spektrum: (CDCl$_3$, TMS als interner Standard)

δ = 1.50 (m) 6 H,
2.12 (m) 2 H,
2.38 (m) 4 H,
2.45 (s) 3 H,
3.45 (s) 2 H,
3.52 (t) 2 H,
4.10 (t) 2 H,
5.5 - 6.3 (breit) 2 H (austauschbar mit $D_2O$)
6.7 - 7.4 (m) 4 H ppm.

Beispiel 7

2-[3-[3-(Piperidinomethyl)phenoxy]propylamino]-5-(2-nitro-benzolsulfenamido)-1,3,4-thiadiazol

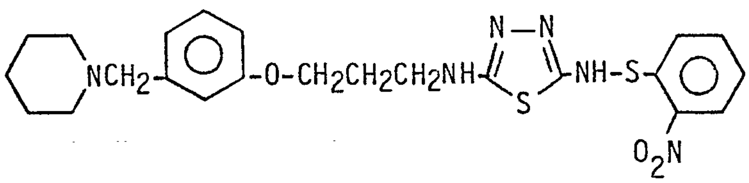

Die Herstellung erfolgt analog Beispiel 5 aus $N^2$-[3-[3-(Piperidinomethyl)phenoxy]propyl]-1,3,4-thiadiazol-2,5-diamin und 2-Nitrobenzolsulfensäurechlorid.

Gelbe Kristalle vom Schmelzpunkt 80 - 83° C

Rf = 0.39 ($Al_2O_3$ / Methylenchlorid / MeOH 95:5)

$C_{23}H_{28}N_6O_3S_2$ (500)

$^1$H-NMR-Spektrum: (d$_6$-DMSO, TMS als interner Standard)

$\delta$ = 1.43 (m) 6 H,
1.98 (m) 2 H,
2.33 (m) 4 H,
3.34 (m) 2 H,
3.39 (s) 2 H,
4.03 (t) 2 H,
6.7 - 8.1 (m) 8 H,
8.42 (dd) 1 H,
9.53 (breit) 2 H (austauschbar mit $D_2O$) ppm.

0197189

Beispiel 8

2-(Pyridin-2-sulfenamido)-5-[3-[3-(3-methylpiperidin-1-ylmethyl)phenoxy]propylamino]-1,3,4-thiadiazol

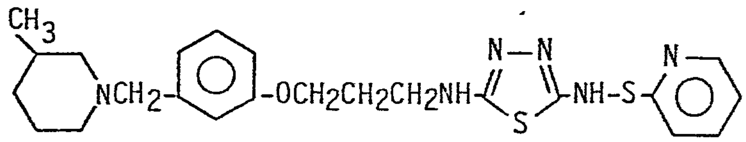

Die Herstellung erfolgt analog Beispiel 5 aus $N^2$-[3-[3-(3-Methylpiperidin-1-ylmethyl)phenoxy]propyl]-1,3,4-thiadiazol-2,5-diamin und 2-Pyridinsulfensäurechlorid.

Hellgelbe Kristalle vom Schmelzpunkt 118° C

Rf = 0.28 ($Al_2O_3$ / Essigsäureethylester / MeOH 90:10)

$C_{23}H_{30}N_6OS_2$ (471)

$^1$H-NMR-Spektrum: d$_6$-DMSO, TMS als interner Standard)

$\delta$ = 0.82 (m) 4 H,
1.3 - 2.3 (m) 8 H,
2.67 (m) 2 H,
3.37 (m) 2 H,
3.39 (s) 2 H,
4.03 (t) 2 H,
6.89 (m) 3 H,
7.27 (m) 4 H (1 H austauschbar mit $D_2O$),
7.87 (m) 1 H,
8.53 (m) 1 H,
9.3 (breit) 1 H (austauschbar mit $D_2O$)
ppm.

Beispiel 9



2-[3-[3-(1-Pyrrolidinomethyl)phenoxy]propylamino]-5-(2-pyridinsulfen-amido)-1,3,4-thiadiazol

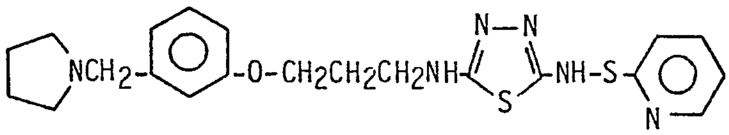

Die Herstellung erfolgt analog Beispiel 5 aus $N^2$-[3-[3-(1-Pyrrolidinomethyl)phenoxy]propyl]-1,3,4-thiadiazol-2,5-diamin und 2-Pyridinsulfensäurechlorid.

Farblose Kristalle vom Schmelzpunkt 122° C

Rf = 0.32 ($Al_2O_3$ / Essigsäureethylester / MeOH 90:10)

| | | | | |
|---|---|---|---|---|
| $C_{21}H_{26}N_6OS_2$ (443) | Ber.: | C 56.99 | H 5.92 | N 18.99 |
| | Gef.: | C 56.97 | H 5.99 | N 19.08 |

$^1$H-NMR-Spektrum: (d$_6$-DMSO, TMS als interner Standard)

$\delta$ = 1.69 (m) 4 H,
1.97 (m) 2 H,
2.45 (m) 4 H,
3.33 (m) 2 H,
3.57 (s) 2 H,
4.03 (t) 2 H,
6.7 - 7.5 (m) 7 H, (1 H austauschbar mit $D_2O$),
7.7 - 8.0 (m) 1 H,
8.50 (m) 1 H,
8.7 - 9.8 (breit) 1 H (austauschbar mit $D_2O$) ppm.

0197189

## Beispiel 10

2-[3-[3-(1-Hexamethyleniminomethyl)phenoxy]propylamino]-5-(2-pyridinsulfenamido)-1,3,4-thiadiazol

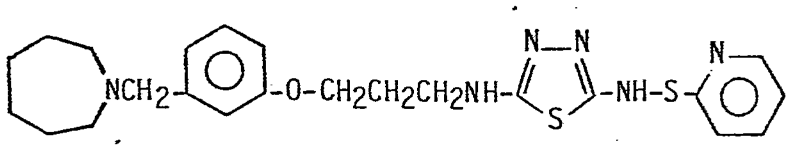

Die Herstellung erfolgt analog Beispiel 5 aus $N^2$-[3-[3-(1-Hexamethyleniminomethyl)phenoxy]propyl]-1,3,4-thiadiazol-2,5-diamin und 2-Pyridinsulfensäurechlorid.

Farblose Kristalle vom Schmelzpunkt 128° C

Rf = 0.34 ($Al_2O_3$ / Essigsäureethylester / MeOH 90:10)

| | | | | |
|---|---|---|---|---|
| $C_{23}H_{30}N_6OS_2$ (471) | Ber.: | C 58.70 | H 6.43 | N 17.86 |
| | Gef.: | C 58.80 | H 6.49 | N 18.08 |

$^1$H-NMR-Spektrum: (d$_6$-DMSO, TMS als interner Standard)

$\delta$ = 1.59 (s, breit) 8 H,
1.97 (m) 2 H,
2.57 (m) 4 H,
3.33 (m) 2 H,
3.60 (s) 2 H,
4.03 (t) 2 H,
6.7 - 7.4 (m) 7 H (1H austauschbar mit $D_2O$),
7.7 - 8.0 (m) 1 H,
8.5 (m) 1 H,
9.3 (breit) 1 H (austauschbar mit $D_2O$) ppm.

Beispiel 11

2-[4-[3-(Piperidinomethyl)phenoxy]butylamino]-5-(2-pyridinsulfenamido)-1,3,4-thiadiazol

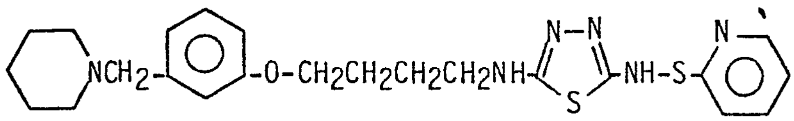

Die Herstellung erfolgt analog Beispiel 5 aus $N^2$-[4-[3-(Piperidinomethyl)phenoxy]butyl]-1,3,4-thiadiazol-2,5-diamin und 2-Pyridinsulfensäurechlorid.

Farblose Kristalle vom Schmelzpunkt 121 - 123° C

Rf = 0.24 ($Al_2O_3$ / Essigsäureethylester / MeOH 90:10)

| | | | | |
|---|---|---|---|---|
| $C_{23}H_{30}N_6OS_2$ (471) | Ber.: | C 58.70 | H 6.43 | N 17.86 |
| | Gef.: | C 58.54 | H 6.50 | N 17.46 |

$^1$H-NMR-Spektrum: ($d_6$-DMSO, TMS als interner Standard)

$\delta$ = 1.45 (m) 6 H,
1.73 (m) 4 H,
2.35 (m) 4 H,
3.26 (m) 2 H,
3.43 (s) 2 H,
4.00 (m) 2 H,
6.8 - 7.45 (m) 7 H, (1 H austauschbar mit $D_2O$),
7.87 (m) 1 H,
8.55 (m) 1 H,
8.8 - 9.8 (breit) 1 H (austauschbar mit $D_2O$) ppm.

Beispiel 12

2-[4-[3-(Piperidinomethyl)phenoxy]butylamino]-5-(4-toluolsulfenamido)-1,3,4-thiadiazol

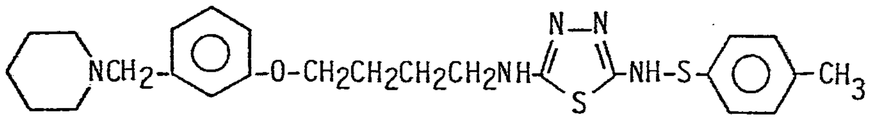

Die Herstellung erfolgt analog Beispiel 5 aus $N^2$-[4-[3-(Piperidinomethyl)phenoxy]butyl]-1,3,4-thiadiazol-2,5-diamin und p-Tolylsulfensäurechlorid.

Farblose Kristalle vom Schmelzpunkt 124 - 125° C

Rf = 0.35 ($Al_2O_3$ / Essigsäureethylester / MeOH 95:5)

$C_{25}H_{33}N_5OS_2$ (484)

| | |
|---|---|
| $^1$H-NMR-Spektrum: (d$_6$-DMSO, TMS als interner Standard) | $\delta$ = 1.1 - 2.0 (m) 10 H,<br>2.0 - 2.45 (m) 7 H,<br>3.2 (m) 2 H,<br>3.38 (s) 2 H,<br>3.96 (m) 2 H,<br>6.7 - 7.4 (m)<br>7.27 (s) 9 H, (1H austauschbar mit $D_2O$),<br>9.15 (breit) 1 H (austauschbar mit $D_2O$)<br>ppm. |

Beispiel 13

2-[4-[3-(Piperidinomethyl)phenoxy]butylamino]-5-cyclohexansulfenamido-1,3,4-thiadiazol

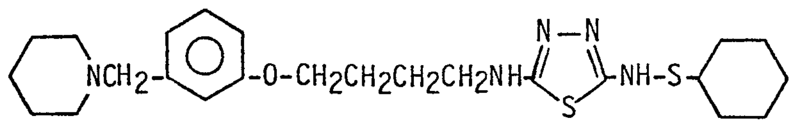

Die Herstellung erfolgt analog Beispiel 5 aus $N^2$-[4-[3-(Piperidinomethyl)phenoxy]butyl]-1,3,4-thiadiazol-2,5-diamin und Cyclohexylsulfensäurechlorid.

Zähes Öl

Rf = 0.54 ($Al_2O_3$ / Essigsäureethylester / MeOH 95:5)

$C_{24}H_{37}N_5OS_2$ (476)

| | |
|---|---|
| $^1$H-NMR-Spektrum: (CDCl$_3$, TMS als interner Standard) | $\delta$ = 1.0 - 2.1 (m) 20 H,<br>2.37 (m) 4 H,<br>2.92 (m) 1 H,<br>3.32 (m) 2 H,<br>3.43 (s) 2 H,<br>3.97 (m) 2 H,<br>5.6 - 6.6 (breit) 2 H (austauschbar mit $D_2O$),<br>6.7 - 7.4 (m) 4 H ppm. |

Beispiel 14

2-[4-[3-(Piperidinomethyl)phenoxy]but-2-enylamino]-5-pyridin-2-sulfenamido-1,3,4-thiadiazol

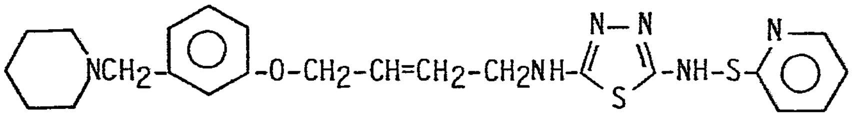

Die Herstellung erfolgt analog Beispiel 5 aus $N^2$-[4-[3-(Piperidinomethyl)phenoxy]-but-2-enyl]-1,3,4-thiadiazol-2,5-diamin und 2-Pyridinsulfensäurechlorid.

Farblose Kristalle vom Schmelzpunkt 129 - 130° C

Rf = 0.54 ($Al_2O_3$ / Essigsäureethylester / MeOH 80:20)

| | | | | |
|---|---|---|---|---|
| $C_{23}H_{28}N_6OS_2$ (469) | Ber.: | C 58.95 | H 6.02 | N 17.93 |
| | Gef.: | C 58.90 | H 6.03 | N 17.94 |

$^1$H-NMR-Spektrum: ($d_6$-DMSO, TMS als interner Standard)

$\delta$ = 1.42 (m) 6 H,
2.32 (m) 4 H,
3.40 (s) 2 H,
3.87 (m) 2 H,
4.53 (m) 2 H,
5.93 (m) 2 H,
6.89 (m) 3 H,
7.1 - 7.5 (m) 4 H (1 H austauschbar mit $D_2O$),
7.7 - 8.0 (m) 1 H,
8.50 (m) 1 H,
9.0 - 9.7 (breit) 1 H (austauschbar mit $D_2O$) ppm.

# PATENTANSPRÜCHE

1. 1,3,4-Thiadiazolderivate der allgemeinen Formel I

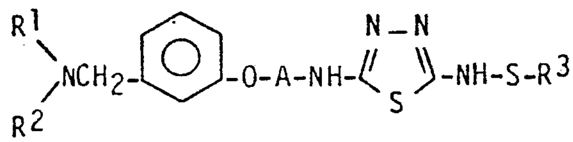

(I)

in der $R^1$ und $R^2$, die gleich oder verschieden sein können, jeweils für Wasserstoff, lineares $C_1$-$C_{10}$-Alkyl, Polycycloalkyl, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen gegebenenfalls ein- oder zweifach mit einer $C_1$-$C_3$-Alkylgruppe substituierten 5- bis 10gliedrigen stickstoffhaltigen alicyclischen, heterocyclischen Ring bilden, A die Gruppierung $-(CH_2)_n-$ oder $-CH_2-CH=CH-CH_2-$ bedeutet, wobei n den Wert 3 oder 4 hat, $R^3$ für lineares oder verzweigtkettiges $C_1$-$C_6$-Alkyl oder $C_5$-$C_6$-Cycloalkyl oder für unsubstituiertes oder ein- bis dreifach substituiertes Aryl oder Heteroaryl steht, sowie die physiologisch annehmbaren Salze davon.

2. Verbindungen nach Anspruch 1, dadurch gekennzeichnet, daß $R^3$ für lineares oder verzweigtkettiges $C_1$-$C_6$-Alkyl oder $C_5$-$C_6$-Cycloalkyl steht.

3. Verbindungen nach Anspruch 1, dadurch gekennzeichnet, daß $R^3$ für unsubstituiertes oder ein- bis dreifach substituiertes Aryl steht.

4. Verbindungen nach Anspruch 1, dadurch gekennzeichnet, daß $R^3$ für einen Pyridin- oder Pyrimidinring steht.

5. Verbindungen nach den Ansprüchen 1 bis 4, dadurch gekennzeichnet, daß $R^1$ und $R^2$ zusammen mit dem Stickstoffatom, an das sie gebunden sind, einen Pyrrolidin-, Piperidin- oder Homopiperidinring

bilden und daß A für die Gruppierung $-(CH_2)_3-$ oder $-CH_2-CH=CH-CH_2-$ steht.

6. 2-[3-[3-(Piperidinomethyl)phenoxy]propylamino]-5-pyridin-2-sulfenamido-1,3,4-thiadiazol und die physiologisch annehmbaren Salze davon.

7. 2-[4-[3-(Piperidinomethyl)phenoxy]butylamino]-5-cyclohexansulfenamido-1,3,4-thiadiazol und die physiologisch annehmbaren Salze davon.

8. 2-(Pyridin-2-sulfenamido)-5-[3-[3-(3-methylpiperidin-1-yl-methyl)-phenoxy]propylamino]-1,3,4-thiadiazol und die physiologisch annehmbaren Salze davon.

9. 2-[4-[3-(Piperidinomethyl)phenoxy]butylamino]-5-(2-pyridinsulfenamido)-1,3,4-thiadiazol und die physiologisch annehmbaren Salze davon.

10. Verfahren zur Herstellung von 1,3,4-Thiadiazolderivaten nach den Ansprüchen 1 bis 6, dadurch g e k e n n z e i c h n e t , daß man

a) eine Verbindung der allgemeinen Formel II

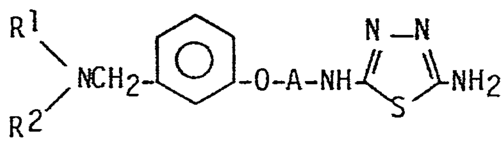

(II)

in der $R^1$, $R^2$ und A die in Anspruch 1 angegebenen Bedeutungen besitzen, mit einem Sulfenylhalogenid der allgemeinen Formel III

$$R^3\text{-S-Hal} \qquad \text{(III)}$$

worin Hal für ein Chlor- oder für ein Bromatom steht und $R^3$ die in Anspruch 1 angegebene Bedeutung hat, zu einer Verbindung der allgemeinen Formel I umsetzt oder daß man

b) eine Verbindung der allgemeinen Formel IV

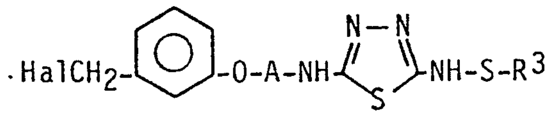 (IV)

worin Hal für ein Chlor- oder für ein Bromatom steht, A und $R^3$ die in Anspruch 1 angegebenen Bedeutungen besitzen, mit einem Amin der allgemeinen Formel V

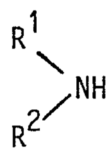 (V)

zu einer Verbindung der allgemeinen Formel I umsetzt

und daß man gegebenenfalls die gemäß a) oder b) erhaltene Verbindung in ihr physiologisch annehmbares Salz umwandelt.

11. Arzneimittel, dadurch g e k e n n z e i c h n e t , daß es eine Verbindung nach den Ansprüchen 1 bis 6 und mindestens einen inerten, pharmazeutisch annehmbaren Träger oder ein inertes, pharmazeutisch annehmbares Verdünnungsmittel enthält.

# PATENTANSPRÜCHE

## Verfahrensansprüche für AT

1. Verfahren zur Herstellung von 1,3,4-Thiadiazolderivaten der allgemeinen Formel I

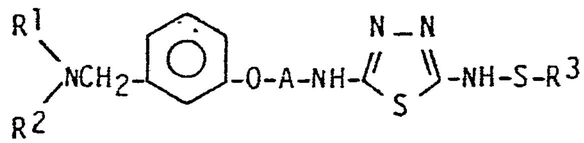

(I)

in der $R^1$ und $R^2$, die gleich oder verschieden sein können, jeweils für Wasserstoff, lineares $C_1$-$C_{10}$-Alkyl, Polycycloalkyl, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen gegebenenfalls ein- oder zweifach mit einer $C_1$-$C_3$-Alkylgruppe substituierten 5- bis 10gliedrigen stickstoffhaltigen alicyclischen, heterocyclischen Ring bilden, A die Gruppierung $-(CH_2)_n-$ oder $-CH_2-CH=CH-CH_2-$ bedeutet, wobei n den Wert 3 oder 4 hat, $R^3$ für lineares oder verzweigtkettiges $C_1$-$C_6$-Alkyl oder $C_5$-$C_6$-Cycloalkyl oder für unsubstituiertes oder ein- bis dreifach substituiertes Aryl oder Heteroaryl steht, sowie der physiologisch annehmbaren Salze davon, dadurch gekennzeichnet, daß man

a) eine Verbindung der allgemeinen Formel II

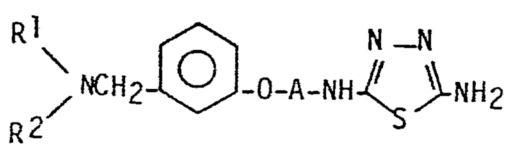

(II)

in der $R^1$, $R^2$ und A die oben angegebenen Bedeutungen besitzen, mit einem Sulfenylhalogenid der allgemeinenFormel III

$$R^3\text{-S-Hal} \qquad \text{(III)}$$

worin Hal für ein Chlor- oder für ein Bromatom steht und $R^3$ die oben angegebene Bedeutung hat, zu einer Verbindung der allgemeinen Formel

AT 0197189

I umsetzt oder daß man

b) eine Verbindung der allgemeinen Formel IV

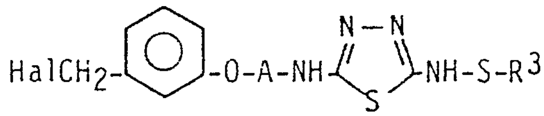

(IV)

worin Hal für ein Chlor- oder für ein Bromatom steht, A und $R^3$ die oben angegebenen Bedeutungen besitzen, mit einem Amin der allgemeinen Formel V

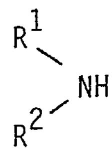

(V)

zu einer Verbindung der allgemeinen Formel I umsetzt

und daß man gegebenenfalls die gemäß a) oder b) erhaltene Verbindung in ihr physiologisch annehmbares Salz umwandelt.

2. Verfahren nach Anspruch 1 zur Herstellung von Verbindungen, bei denen $R^3$ für lineares oder verzweigtkettiges $C_1$-$C_6$-Alkyl oder $C_5$-$C_6$-Cycloalkyl steht.

3. Verfahren nach Anspruch 1 zur Herstellung von Verbindungen, bei denen $R^3$ für unsubstituiertes oder ein- bis dreifach substituiertes Aryl steht.

4. Verfahren nach Anspruch 1 zur Herstellung von Verbindungen, bei denen $R^3$ für einen Pyridin- oder Pyrimidinring steht.

5. Verfahren nach den Ansprüchen 1 bis 4 zur Herstellung von Verbindungen, bei denen $R^1$ und $R^2$ zusammen mit dem Stickstoffatom, an das sie gebunden sind, einen Pyrrolidin-, Piperidin- oder Homopiperidinring bilden und bei denen A für die Gruppierung $-(CH_2)_3-$ oder $-CH_2-CH=CH-CH_2-$ steht.

6. Verfahren nach Anspruch 1 zur Herstellung von 2-[3-[3-(Piperidinomethyl)phenoxy]propylamino]-5-pyridin-2-sulfenamido-1,3,4-thiadiazol und der physiologisch annehmbaren Salze davon.

7. Verfahren nach Anspruch 1 zur Herstellung von 2-[4-[3-(Piperidinomethyl)phenoxy]butylamino]-5-cyclohexansulfenamido-1,3,4-thiadiazol und der physiologisch annehmbaren Salze davon.

8. Verfahren nach Anspruch 1 zur Herstellung von 2-(Pyridin-2-sulfenamido)-5-[3-[3-(3-methylpiperidin-1-yl-methyl)phenoxy]propylamino]-1,3,4-thiadiazol und der physiologisch annehmbaren Salze davon.

9. Verfahren nach Anspruch 1 zur Herstellung von 2-[4-[3-(Piperidinomethyl)phenoxy]butylamino]-5-(2-pyridinsulfenamido)-1,3,4-thiadiazol und der physiologisch annehmbaren Salze davon.